# EUROPEAN PATENT APPLICATION

(11) **EP 2 878 976 A1**
(43) Date of publication of application: **03.06.2015**
(21) Application number: 13195217.8
(22) Date of filing: 30.11.2013
(51) Int. Cl.: G01T 1/29, H01J 47/02

(54) **Device and method for radiation dosimetry based on a ionization chamber**

(71) Applicant: ION BEAM APPLICATIONS S.A., 1348 Louvain-la-Neuve (BE)
(72) Inventor: Menichelli, David, 90537 Feucht (DE); Friedl, Friedrich, 90559 Burgthann (DE)
(74) Representative: Wauters, Davy Erik Angelo

(57) **Abstract**

The present invention relates to a dosimetry device (10) for determining a spatial distribution of a quantity of radiation incident on the dosimetry device. The device comprises a segmented electrode assembly (12) comprising an electrically nonconducting substrate (13) having a plurality of electrode elements (14) provided thereon. The device further comprises an electrically conducting sheet (16) comprising a perimetral protrusion (17) arranged in mechanical contact with the segmented electrode assembly (14) such as to define at least one ionization chamber cavity (18) between the segmented electrode assembly (14) and the electrically conducting sheet (16). The device also comprises a voltage applying means (28) for applying a voltage difference between the electrically conducting sheet (16) and the plurality of electrode elements (14) and a routing means (25) for routing a plurality of ionization currents corresponding to the plurality of electrode elements (14) to a processing means.

## Description

### Field of the invention

The invention relates to the field of radiation dosimetry. More specifically it relates to a dosimetry device for determining a spatial distribution of a quantity of radiation such as an ionization chamber array for quality assurance and/or online verification in radiotherapy or particle therapy, e.g. proton or carbon therapy. The invention also provides a related system and method for determining a spatial distribution of a quantity of radiation.

### Background of the invention

In radiation dosimetry, dosimeters such as ionization chambers are used for performing measurements in order to calculate the radiation dose received by matter, e.g. tissue, due to the exposure to indirect and/or direct ionizing radiation. Radiation dosimetry is particularly important in the fields of medical physics, e.g. for monitoring the required treatment dose and any collateral dose. Air-filled ionization chambers can be considered as the gold standard for dosimetric detection in medical physics due to similarity between radiation interaction with air and organic tissues.

For radiation therapy, arrays of ionization chambers may be particularly useful for determining or monitoring a spatial distribution of a radiation field, e.g. a high-energy photon beam shaped to correspond to a target dose distribution in a patient. Ionization chamber arrays as known in the art may comprise for example air-vented cubic ionization chambers, air-vented cylindrical chambers, covering a squared area with a pitch of 5 mm or higher, e.g. a pitch of 7.6 mm or a pitch of 10 mm. The number of ionization chambers in such array may for example be 251 or higher, e.g. 729 chamber elements, or even higher, e.g. 1020 chamber elements.

An ionization chamber array as known in the art may comprise a plurality of individual ionization chambers placed side by side in an array geometry, for example as described by Martell et al. in "A flatness and calibration monitor for accelerator photon and electron beams", International Journal of Radiation Oncology- Biology-Physics 12, pages 271-275. However, manufacturing such composite arrays of ionization chambers with high spatial resolution and/or a large number of pixel elements, e.g. hundreds or even thousands of pixels, may be unpractical and expensive.

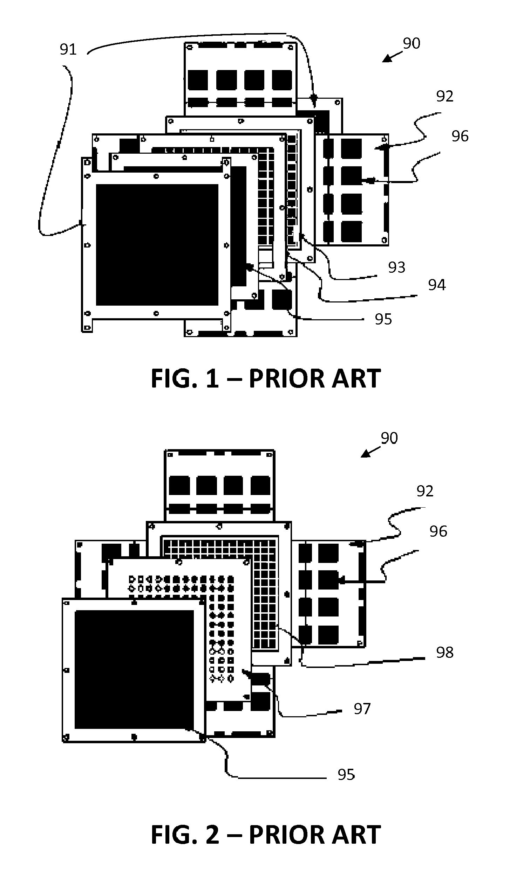

Other ionization chamber arrays known in the art, for example as disclosed in United States patent No. US 8,044,364, may comprise a segmented planar electrode and a common planar electrode arranged in a parallel plane geometry, in which the distance between the electrodes is fixed by an external insulating frame running along the array perimeter, such that a large air-filled cavity is formed between the electrodes. An example of this type of prior-art ionization chamber array 90 is shown in FIG. 1. The prior-art ionization chamber array 90 may for example comprise two mylar frames 91 enclosing an electronic board 92 with very large scale integration chips 96 for signal acquisition and processing, a segmented planar electrode with pixel elements 93 a common planar cathode electrode 95 and a gap frame 94 for spacing the segmented electrode and the common electrode.

This design enables a more efficient manufacture of the ionization chamber array compared to a composite array of individual ionization chambers, however such integrated ionization chamber array may be prone to mechanical instability. The top electrode is supported on its external edge by the spacer frame, and therefore can bend in the central region, which may be especially disadvantageous in the case of a large area array and/or in applications which requires a particularly thin electrode. Furthermore, mechanical instability may couple to ambient vibrations, such that a noise component can be induced thereby in the measured signal, e.g. a signal component similar to microphone transduction. Another disadvantage of this integrated design may be blurring of the measured spatial dosimetric distribution, e.g. the generated dosimetric image, due to secondary electrons generated by the interaction of the incident beam with the medium in the cavity and/or the top electrode walls. In the common cavity, secondary electrons may freely migrate, even in a direction transverse to the beam. Therefore, photons or high-energy particles entering in the cavity volume portion corresponding to a certain pixel may generate a signal in an adjacent one, thus leading to image blurring.

Yet other ionization chamber arrays are known in the art in which a segmented electrode and a common electrode are separated by an insulating layer with holes. An example thereof has been described in international patent application WO 2012/168601 and in patent no. US7476867. Each hole may be centered on a pixel, thus defining the volume of the corresponding ionization chamber. An example of this design is illustrated in FIG. 2. The insulating layer with holes 97 in this type of ionization chamber array may advantageously provide a good mechanical support for the top electrode, and may inhibit diffusion of secondary electrons between pixel elements. Nevertheless, the material of the insulating layer may be damaged by incident radiation. Particularly, electrostatic charge may accumulate in the insulating material, where the lack of charge neutrality may furthermore influence the electric field inside the cavities. This may lead to variations in pixel sensitivity over time, and thus may limit long term stability.

### Summary of the invention

It is an object of embodiments of the present invention to provide good means and methods for determining a spatial distribution of photon or particle radiation.

It is an advantage of embodiments of the present invention that a high spatial resolution and/or a large number of pixel elements can be easily and cost-efficiently manufactured.

It is an advantage of embodiments of the present invention that good mechanical stability can be achieved in an ionization chamber array. It is a further advantage of such embodiments that no substantial microphone transduction noise component is present in the radiation measurement signal acquired by a device or method according to embodiments.

It is an advantage of embodiments of the present invention that little blurring of the measured spatial dosimetric distribution due to inter-pixel diffusion of secondary electrons in the cavity volume, and therefore little blurring of the generated dosimetric image, occurs.

It is an advantage of embodiments of the present invention that small ionization chambers may be provided in a dosimetric array, e.g. providing high spatial resolution and improved performances in high dose rate condition.

It is an advantage of embodiments of the present invention that good long term stability of a dosimetry device according to embodiments can be achieved.

It is an advantage of particular embodiments of the present invention that even better spatial resolution and/or angular dependence can be achieved by coupling two common electrodes to the top and bottom surfaces of the same PCB. The above objective is accomplished by a method and device according to the present invention.

The present invention relates to a dosimetry device for determining a spatial distribution of a quantity of radiation incident on the dosimetry device, the dosimetry device comprising
a segmented electrode assembly comprising an electrically non-conducting substrate having a plurality of electrode elements provided thereon, and
an electrically conducting sheet comprising a perimetral protrusion arranged in mechanical contact with said segmented electrode assembly such as to define at least one ionization chamber cavity between the segmented electrode assembly and the electrically conducting sheet, the at least one ionization chamber cavity being filled with a fluid, and
a voltage applying means for applying a voltage difference between the electrically conducting sheet and the plurality of electrode elements and a routing means for routing a plurality of ionization currents corresponding to the plurality of electrode elements to a processing means.

The segmented electrode assembly may comprise a printed circuit board having said plurality of electrode elements defined thereon.

The plurality of electrode elements may be printed on the printed circuit board using a carbon-based material.

The electrically conducting sheet may comprise a material with a low atomic number such as carbon , conductive plastic and/or aluminum.

The fluid medium may comprise air.

The electrically conducting sheet may comprise at least one further protrusion to further define the at least one additional ionization chamber cavity such that the electrically conducting sheet may comprise a plurality of concave surface regions corresponding to the plurality of electrode elements.

The plurality of electrode elements may be arranged along at least a one dimensional or two dimensional regular grid on said electrically non-conducting substrate.

The plurality of electrode elements may extend into the at least one ionization chamber cavity and the plurality of electrode elements may be adapted in shape for tailoring the electric field in said at least one ionization chamber cavity.

The segmented electrode assembly may comprise a plurality of guard electrodes, each guard electrode being arranged around a corresponding electrode element of the plurality of electrode elements.

The plurality of electrode elements may comprise a first plurality of electrode elements provided on a first major surface of the electrically non-conducting substrate and a second plurality of electrode elements provided on a second major surface of the electrically non-conducting substrate. The perimetral protrusion may be arranged in mechanical contact with the first major surface of the electrically non-conducting substrate. The dosimetry device further may comprise a second electrically conducting sheet comprising a perimetral protrusion arranged in mechanical contact with the second major surface of the electrically non-conducting substrate such as to define at least one ionization chamber cavity between the segmented electrode assembly and the electrically conducting sheet.

The first plurality of electrode elements and the second plurality of electrode elements may be arranged according to the same pattern on respectively the first major surface and the second major surface, and the device may comprise means for electrically connecting each electrode of the first plurality of electrodes to the corresponding electrode of the second plurality of electrodes.

The first plurality of electrode elements and the second plurality of electrode elements may be arranged according to the same pattern, the first plurality of electrode elements being spatially offset in the plane of the substrate with respect to the second plurality of electrode elements.

The dosimetry device may further comprise at least one further segmented electrode assembly comprising a further electrically non-conducting substrate having a further plurality of electrode elements provided thereon, and at least one further electrically conducting sheet comprising a further perimetral protrusion arranged in mechanical contact with said at least one further segmented electrode assembly such as to define at least one further ionization chamber cavity between the at least one further segmented electrode assembly and the at least one further electrically conducting sheet.

The present invention also relates to a dosimetry system comprising a dosimetry device as described above and a processing means for receiving the plurality of ionization currents corresponding to the plurality of electrode elements via the routing means, the processing means being adapted for determining a spatial distribution of radiation dose, radiation dose rate or radiation fluence corresponding to a radiation quantity incident on the dosimetry device taking into account the plurality of ionization currents.

The present invention also relates to a method for determining a spatial distribution of a quantity of radiation, the method comprising:
applying a voltage difference between a plurality of electrode elements provided on an electrically non-conducting substrate and an electrically conducting sheet having a perimetral protrusion arranged in mechanical contact with the electrically non-conducting substrate, the perimetral protrusion defining at least one ionization chamber cavity between the electrically non-conducting substrate and the electrically conducting sheet, and
determining a plurality of ionization currents corresponding to the plurality of electrode elements generated by interaction of radiation within a fluid medium in said at least one ionization chamber cavity.

The method may further comprise positioning the electrically non-conducting substrate and the electrically conducting sheet between a source of the radiation and a target, such that the radiation travels through the at least one ionization chamber cavity prior to delivering the radiation to the target.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

FIG. 1 shows a prior-art ionization chamber array comprising a pixelated anode planar electrode and a cathode planar electrode, separated by a gap frame.
FIG. 2 shows a prior-art ionization chamber array comprising a pixelated anode planar electrode and a cathode planar electrode, separated by an insulating layer with holes.
FIG. 3 shows a device according to a first embodiment of the present invention.
FIG. 4 shows a device according to a second embodiment of the present invention.
FIG. 5 shows a device according to a third embodiment of the present invention.
FIG. 6 shows a first exemplary device having a pixel electrode element extending into the ionization cavity according to embodiments of the present invention.
FIG. 7 shows a second exemplary device having a pixel electrode element extending into the ionization cavity according to embodiments of the present invention.
FIG. 8 shows a first exemplary device comprising two conductive sheets on opposite surfaces of the segmented electrode assembly according to embodiments of the present invention.

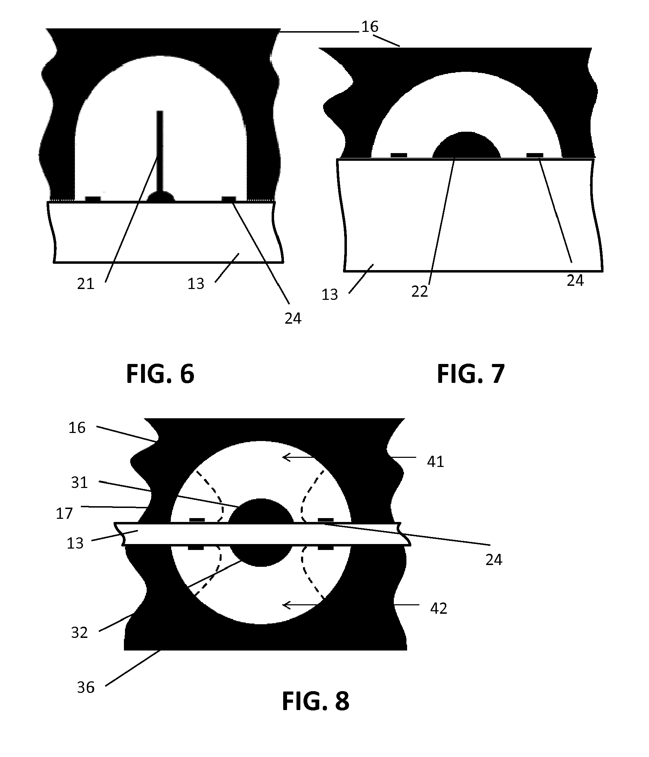

FIG. 8 shows a second exemplary device comprising two conductive sheets on opposite surfaces of the segmented electrode assembly according to embodiments of the present invention.

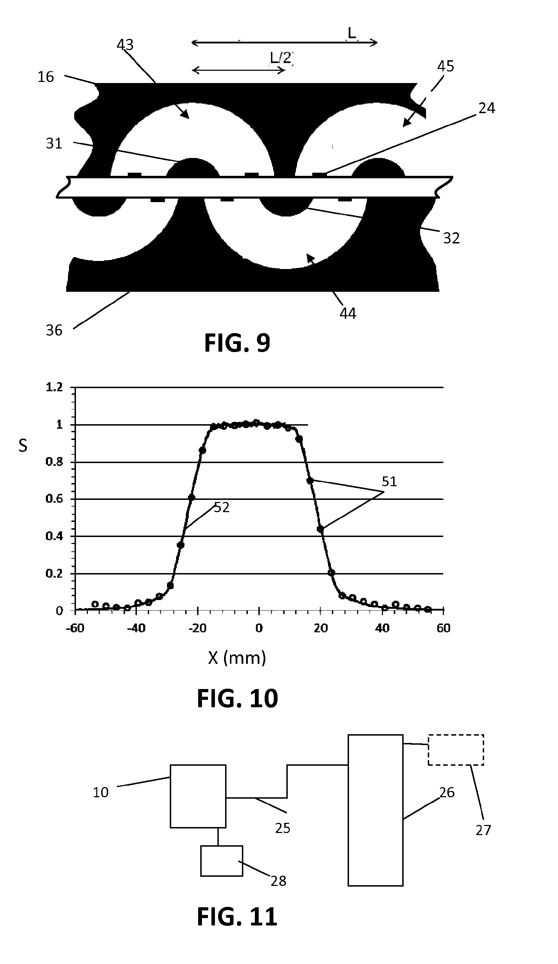

FIG. 10 shows an experimental comparison of a radiation beam profile acquired with a device according to embodiments of the present invention and with a reference detector.
FIG. 11 shows a system according to embodiments of the present invention.

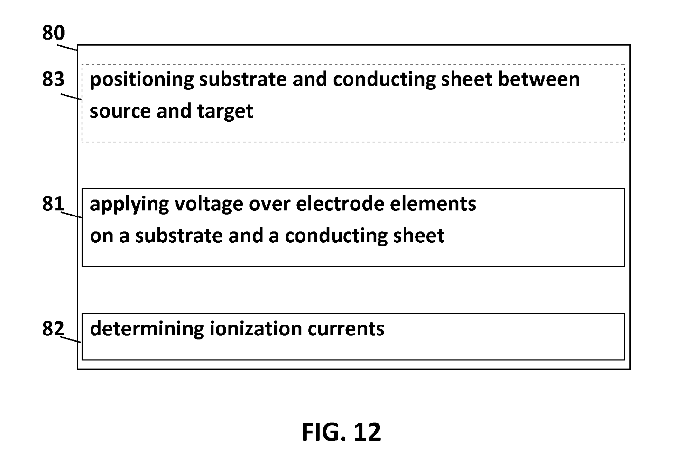

FIG. 12 shows a method according to embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a dosimetry device for determining a spatial distribution of a quantity of radiation incident on the dosimetry device. The dosimetry device may also be referred to as a radiation dosimeter or as an ionization chamber array. The radiation incident on the dosimetry device may for example comprise uncharged radiation, e.g. high energetic photon radiation or neutron radiation, and/or charged radiation, e.g. proton radiation, electron radiation, charged hadron radiation or ion radiation. Determining the spatial distribution of a quantity of the radiation may thus comprise determining in a plurality of regions in space, e.g. regions distributed along a line, on a plane or in a volume, a measure associated with the radiation. Such measure may for example comprise a radiation dose, dose rate, or fluence, e.g. a measure for kinetic energy released per unit mass (kerma).The device according to embodiments of the present invention may for example be suitable for determining a spatial radiation dose distribution generated by for example an incident photon beam comprising energies in the range of 1 MeV to 20 MeV, or an incident proton beam comprising energies in the range of 50 MeV to 250 MeV. A device according to embodiments may advantageously achieve a high spatial resolution, e.g. may have an effective pixel pitch of less than 5 mm, for example 3.5 mm or even less, for example 2 mm.

The dosimetry device comprises a segmented electrode assembly which comprises an electrically non-conducting substrate having a plurality of electrode elements provided thereon. The dosimetry device further comprises an electrically conducting sheet which comprises a perimetral protrusion arranged in mechanical contact with the segmented electrode assembly such as to define at least one ionization chamber cavity between the segmented electrode assembly and the electrically conducting sheet. The dosimetry device further comprises a voltage applying means for applying a voltage difference between the electrically conducting sheet and the plurality of electrode elements, e.g. for applying a bias potential to the electrically conducting sheet with respect to a pixel element potential. The voltage applying means may be any type of voltage applying means, such as for example a voltage source. The voltage applying means also may comprise a set of electrodes connected to the voltage source for applying a voltage to certain nodes. The dosimetry device also comprises a routing means for routing a plurality of ionization currents corresponding to the plurality of electrode elements to a processing means. Such a routing means may for example correspond with a plurality of electrodes. Different standard and optional features in exemplary embodiments will be discussed in further detail with reference to the drawings.

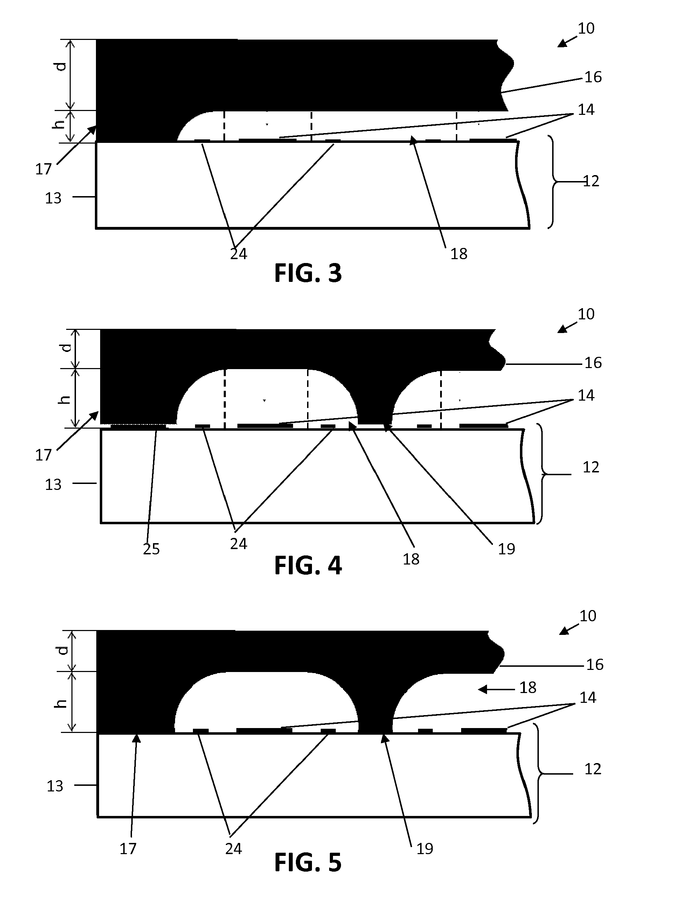

Referring to FIG. 3, a dosimetry device 10 according to a first embodiment of the present invention is shown. This dosimetry device 10 is suitable for determining a spatial distribution of a quantity of radiation incident on the dosimetry device 10.

The dosimetry device 10 comprises a segmented electrode assembly 12. The segmented electrode assembly 12 comprises an electrically non-conducting substrate 13, e.g. a substrate comprising an electrically insulating material, e.g. a plastic substrate, a ceramic substrate, a substrate comprising a fiber-glass based material commonly used for PCB's, a material having a relative permittivity of at least 2.0, for example silicon dioxide, etc.. Particularly, the electrically non-conducting substrate 13 may be a planar electrically non-conducting substrate.

The electrically non-conducting substrate 13 has a plurality of electrode elements 14 provided thereon. Thus, a segmented electrode, e.g. the plurality of electrode elements 14, may be defined on the insulating surface of a planar support, e.g. a major surface of the non-conducing substrate 13.

The plurality of electrode elements 14 may be arranged along at least one straight line on the electrically non-conducting substrate 13. For example, a one-dimensional array of electrode elements 14 may be provided on the electrically non-conducting substrate 13. The plurality of electrode elements 14 may be configured as a grid, e.g. a regular grid such as an equidistantly spaced Cartesian grid, on the electrically non-conducting substrate 13. However, other arrangements of the plurality of electrode elements 14 lie well within the scope of the present invention, for example a polar grid arrangement.

The segmented electrode assembly 12 may also comprise a plurality of guard electrodes 24, e.g. annular guard electrodes, in which each guard electrode is arranged around a corresponding electrode element of the plurality of electrode elements 14. Thus, each of the pixel electrodes may be substantially encircled by a corresponding conductive guard ring, for example the pixel electrodes may be encircled by a conductive guard ring.

The active volume of each pixel within the at least one ionization chamber cavity, e.g. within the air-filled cavities, may be defined by the lines of electric field. The charge ionized by radiation close to the guard electrodes will flow through this electrode, thus reducing the blurring of dosimetric images formed by the device..

The plurality of electrode elements 14, e.g. the pixel electrodes, may provide charge readout for the individual pixels, e.g. by supplying an ionization current via the routing means to a processing means. Each pixel electrode may be surrounded by a guard electrode kept at the same potential to avoid the flow of surface current from the conducting sheet, e.g. the common electrode, to pixels. All the guard electrodes may be connected together, e.g. may be maintained at the same electrical potential.

The segmented electrode assembly 12 may comprise a printed circuit board (PCB) having the plurality of electrode elements 14 defined thereon. The plurality of electrode elements, e.g. the pixel electrodes, may be preferably printed on the printed circuit board with a carbon-based material, such that a better quality of dosimetric measurements can be achieved compared to conventional copper printing. Likewise, the plurality of guard electrodes and/or at least a portion of the voltage applying means 28 and/or the routing means 25 (not shown in FIG. 3 but shown schematically in FIG. 11) may be printed with such carbon-based material as well. The latter typically may improve the ionization chamber energy dependence.

The dosimetry device 10 further comprises an electrically conducting sheet 16 which comprises a perimetral protrusion 17 arranged in mechanical contact with the segmented electrode assembly 12 such as to define at least one ionization chamber cavity 18 between the segmented electrode assembly and the electrically conducting sheet. Thus, the electrically conducting sheet 16 may be directly connected, e.g. mechanically connected, to the segmented electrode assembly 12 via at least the perimetral protrusion 17. For example, the electrically conducting sheet 16 may be connected to the segmented electrode assembly 12 by conventional means such as gluing or soldering. Furthermore, small gaps or channels may be defined in the perimetral protrusion in order to allow venting of the ionization chamber cavity, e.g. in the case of an air-filled cavity, without substantially affecting the mechanical stability of the device.

In the conductive material of the electrically conducting sheet 16, electrons may move freely, such that charge trapping induced by radiation can be compensated in the sheet 16. Therefore, embodiments of the present invention may advantageously exhibit good resistance to radiation damage.

The device may also comprise a fluid medium filling the at least one ionization chamber cavity 18, e.g. the at least one ionization chamber cavity 18 may be a gas-filled ionization chamber cavity. For example, the fluid medium may comprise air. However, other media may also be encompassed by the scope of the present invention, for example noble gases or liquids suitable for ionization chambers.

The electrically conducting sheet 16 may be a common planar electrode or common top electrode of a pixelated plane parallel ionization chamber, in which the plurality of electrode elements 14 may form the bottom electrodes corresponding to the common top electrode. The electrically conducting sheet 16 may comprise a graphite material. The electrically conducting sheet 16 may comprise an electrically conductive plastic, e.g. an intrinsically conducting polymer, and/or low atomic weight metals or metal alloys, e.g. aluminum.

Thus, the common top electrode may be advantageously produced from a single slab of conductive material, and the at least one ionization chamber cavity may be formed by carving, milling or machining the surface of the conductive plate facing the segmented electrode assembly. Other processing techniques nevertheless also may be made.

In order to improve the ionization chamber energy dependence, such a material may also have preferably a low atomic number, such as conductive plastic or graphite, in order to reproduce radiation-matter interaction in organic tissues or water. Aluminium, or other light metals or metal alloys, may also be used to achieve a good mechanical accuracy of production. Combinations of such materials may also be used, e.g. homogeneous compounds or mixtures thereof and/or layered structures formed by such materials.

The dosimetry device 10 may also comprise a voltage applying means for applying a voltage difference between the electrically conducting sheet and the plurality of electrode elements 14 and a routing means for routing a plurality of ionization currents corresponding to the plurality of electrode elements 14 to a processing means. For example, the voltage applying means and/or the routing means may comprise conductive paths arranged in, on and/or through the electrically non-conducting substrate, e.g. printed on a printed circuit board. The routing means may comprise multiplexers and/or addressing means for routing the plurality of ionization currents to the processing means. The device 10 may comprise routing means, e.g. printed circuit board connections, wires and/or multiplexing or addressing means as commonly used in the art, the implementation of such routing means requiring mere application of common knowledge and skill. The processing means may be an external processing means, e.g. the routing means may provide a signal output port for connecting the processing means to. The processing means may for example comprise an integrated circuit adapted for processing signals representative of the ionization currents. The processing means may comprise a general purpose processor programmed for processing such signals. The processing means may also be entirely or in part implemented in or on the electrically conducting substrate, e.g. processing of the signals may take place in an integrated circuit provided on the electrically conducting substrate, for example in an edge region of the substrate such that interaction of the incident radiation being measured and the integrated circuit is substantially avoided. Methods for the processing of signals representing ionization currents acquired by ionization chamber arrays are well-known in the art.

In advantageous embodiments, the mechanical connection of the electrically conducting sheet 16 to the segmented electrode assembly 12 via the perimetral protrusion may also provide a conductive path to a portion of the voltage applying means arranged on the electrically non-conducting substrate, such that the connection for applying a voltage difference between the electrically conducting sheet and the plurality of electrode elements 14 does not require a separate wire connection to the electrically conducting sheet 16. For example, the common top electrode may sit on a conductive ring (an example thereof being shown in FIG.4 by conductive ring 25) on the printed circuit board (PCB) which is used to set the potential difference between the common electrode and pixel electrodes. Therefore, in particular embodiments of the present invention the common electrode can be biased through the printed circuit board, while in other embodiments the common electrode can be biased independently with a separate cable. For example, the top electrode may sit directly on the insulating PCB surface. The bias voltage may be provided by a separate cable, directly connected to the top electrode. Therefore, no high voltage difference needs to be present on the PCB, which may help reducing leakage currents in applications in which very small currents have to be detected.

Referring to FIG. 4, a device according to a second exemplary embodiment of the present invention is shown. Here, the electrically conducting sheet 16 may comprise at least one further protrusion 19 to further define the at least one additional ionization chamber cavity 18 such that the electrically conducting sheet 16 comprises a plurality of concave surface regions corresponding to the plurality of electrode elements 14. The at least one further protrusion 19 may extend toward the segmented electrode assembly 12 without establishing mechanical contact between the at least one further protrusion 19 and the segmented electrode assembly 12. Such arrangement may advantageously allow the flow of a fluid medium filling the at least one ionization chamber cavity 18 through the gap space left between the segmented electrode assembly 12 and the at least one further protrusion 19, e.g. for allowing pressure equalisation over the at least one ionization chamber cavity 18, while substantially preventing the migration of secondary electrons generated by incident radiation between the volumes enclosed by adjacent concave surface regions. Thus, such walls delimiting cavities in the top electrode may moderate the lateral motion of secondary electrons and image blurring. These walls may come close to segmented electrode assembly, e.g. the printed circuit board surface, without touching it, such that good electrical insulation between top electrode and the segmented electrode assembly, e.g. the guard electrodes thereon, may be provided. In this way a high electric field can be applied between the pixel electrodes and the common electrode, thus achieving good performance in high-dose-rate applications.

An additional advantage is that the thickness h of the cavities may be independent of the overall thickness h+d of the electrode. More particularly, as the thickness of the cavities can be selected by the processing of the cavity in the electrically conducting sheet, it becomes possible to construct chambers with a very thin active volume, e.g. h smaller than or equal to 1mm, while retaining a good mechanical stability by selecting a sufficiently high thickness d of the remaining part.

In other embodiments, e.g. as shown in FIG. 5, the at least one further protrusion 19 may mechanically contact the segmented electrode assembly 12. This advantageously even further prevents the migration of secondary electrons and further improves the mechanical stability of the device by providing additional mechanical support. Thus, the at least one ionization cavity 18 may comprise a plurality of pixel ionization chambers, e.g. formed by the perimetral protrusion and the at least one further protrusion, such that for example each pixel ionization chamber corresponds to one electrode element of the plurality of electrode elements 14.

The single cavity manufactured within the top electrode shown in FIG. 2 may simplify the construction of the electrically conducting sheet 16, and may simplify the alignment between the top electrode and the pixel array. Furthermore, the lateral spread of secondary electrodes may be negligible if the cavity thickness h is small enough in comparison to pixel pitch.

While in particular embodiments the pixel electrode elements may be substantially flat, e.g. merely obtained by printing, in other embodiments the electrode elements extending into the at least one ionization chamber cavity may provide an additional degree of freedom to tailor the electric field lines, e.g. for increasing the maximum bias and chamber charge collection efficiency. In embodiments according to the present invention, the plurality of electrode elements 14 may extend into the at least one ionization chamber cavity 18. Particularly, the plurality of electrode elements 14 may be adapted in shape for tailoring the electric field in the at least one ionization chamber cavity. For example, as illustrated in FIG. 6, the electrode element, e.g. pixel electrode, may comprise a pin 21 arranged on the surface of the electrically non-conducting substrate 13 facing the electrically conducting sheet 16, e.g. a pin soldered or glued to the printed circuit board surface. In another example, e.g. as illustrated in FIG. 7, the electrode element may comprise a dot of conductive glue 22 arranged on the surface of the electrically non-conducting substrate 13. Thus, the shape of electric field lines in the cavity may be tailored to achieve the highest bias and collection efficiency. An electrode glued or soldered on the PCB may also have different shapes, as will be apparent to the person skilled in the art, as suitable for the specific intended application. For example, a thin cylindrical electrode may be used to distribute lines of electric field in a relatively large electrode, e.g. to increase active volume and sensitivity while preserving electric field strength and charge collection efficiency at high dose rate.

As depicted in FIG. 8, in embodiments of the present invention, the plurality of electrode elements may comprise a first plurality of electrode elements 31 provided on a first major surface of the electrically non-conducting substrate 13 and a second plurality of electrode elements 32 provided on a second major surface of the electrically non-conducting substrate 13. The perimetral protrusion 17 of the electrically conducting sheet 16 may be arranged in mechanical contact with the first major surface of the electrically non-conducting substrate 13. In such embodiments, the dosimetry device may further comprise a second electrically conducting sheet 36 comprising a perimetral protrusion arranged in mechanical contact with the second major surface of the electrically non-conducting substrate 13 such as to define at least one ionization chamber cavity between the segmented electrode assembly 14 and the electrically conducting sheet 16. Thus, two common electrodes, the electrically conducting sheet 16 and the second electrically conducting sheet 36, may be used per segmented electrode assembly 12, e.g. per PCB, to define cavities on its top and bottom surfaces. The electrically conducting sheet 16 and the second electrically conducting sheet 36 may be electrically connected via the voltage applying means, e.g. to supply the same bias voltage to both sheets. Both sheets may be placed in a symmetric position on the two opposing surfaces of the segmented electrode assembly 12, in order to obtain an active volume having a geometry proximate to that of a sphere. Such arrangement may advantageously improve the angular dependence of the detector, e.g. the pixel sensitivity may be substantially equal regardless of the direction of incident radiation. For example the active volume of each pixel may be split in a top half 41 and a bottom half 42.

An alternative arrangement is shown in FIG. 9, in which the first plurality of electrode elements 31 and the second plurality of electrode elements 32 may be provided with a relative spatial offset. Such offset, e.g. a relative offset L/2 of half the pitch L of the first and second plurality of electrode elements, may advantageously be used to improve spatial resolution. This design is particularly suited for producing an array with high spatial resolution. Since the minimum achievable pitch of cavities in the common electrodes and of electrode printing on PCB surfaces may be constrained by technological considerations and costs, a pixel pitch as small as one half of cavity and prints pitch L may be obtained. For example, the active volume for the N'th pixel 43 and the (N+2)'th pixel 45 may be provided on the top side, while the active volume for the (N+1)'th pixel 44 may be provided on the bottom side, e.g. active volumes for spatially consecutive pixel elements may be provided on alternatingly the bottom and the top surface.

In embodiments according to the present invention, the dosimetry device may also comprise at least one further segmented electrode assembly comprising a further electrically non-conducting substrate having a further plurality of electrode elements provided thereon, and at least one further electrically conducting sheet comprising a further perimetral protrusion arranged in mechanical contact with the at least one further segmented electrode assembly such as to define at least one further ionization chamber cavity between the at least one further segmented electrode assembly and the at least one further electrically conducting sheet. Thus, a segmented electrode assembly 12, an electrically conducting sheet 16 and a voltage applying means and/or routing means as described previously may form a module, in which a device according to embodiments may comprise several such modules arranged to cover a wider area in a plane, e.g. for simplifying service operations on the device, or may be arranged in more complex arrangements, e.g. to determine a three-dimensional spatial distribution of radiation, for example by stacking the modules.

In a second aspect, the present invention also relates to a dosimetry system, e.g. as illustrated in FIG. 11. Such dosimetry system comprises a dosimetry device 10 according to the first aspect of the present invention and a processing means 26, e.g. a processor, for receiving the plurality of ionization currents corresponding to the plurality of electrode elements 14 via the routing means 25 of the dosimetry device 10. The processing means 26 is furthermore adapted for, e.g. programmed for, determining a spatial distribution of radiation dose, radiation dose rate or radiation fluence corresponding to a radiation quantity incident on the dosimetry device 10 taking into account the plurality of ionization currents. The dosimetry system may further comprise an output means 27 for outputting data corresponding to or derived from the spatial distribution of radiation dose, radiation dose rate or radiation fluence.

In a third aspect, the present invention also relates to a method 80 for determining a spatial distribution of a quantity of radiation. Referring to FIG. 12, an exemplary method according to embodiments is schematically illustrated. The method 80 comprises applying 81 a voltage difference between a plurality of electrode elements 14 provided on an electrically non-conducting substrate 13 and an electrically conducting sheet 16 having a perimetral protrusion 17 arranged in mechanical contact with the electrically non-conducting substrate 13, in which the perimetral protrusion defines at least one ionization chamber cavity 18 between the electrically non-conducting substrate 13 and the electrically conducting sheet 16. The method 80 further comprises determining 82 a plurality of ionization currents corresponding to the plurality of electrode elements 14 generated by interaction of radiation within a fluid medium in the at least one ionization chamber cavity 18.

The method 80 may also comprise positioning 83 the electrically non-conducting substrate 13 and the electrically conducting sheet 16 between a source of the radiation and a target, such that the radiation travels through the at least one ionization chamber cavity 18 prior to delivering the radiation to the target.

As an example for illustrating embodiments according to the present invention, experimental results achieved with a linear array of 32 pixels according to embodiments is shown in FIG. 10. The detector used for this example, with a structure similar to the design shown in FIG. 4, was composed of a multi-layer PCB, on which pixel and guard electrodes were defined with carbon printing. The top electrode has individual pixel cavities extending close to PCB surface, and is made of conductive plastic (POM). The pixel electrodes were held at ground potential, while applying a 50V bias to the top electrode. The pixel pitch was 3.5mm and thickness of the air cavities h was approximately equal to 1mm. This pitch may presently be the smallest or one of the smallest achieved in air-filled ionization chamber arrays. In FIG. 10, the measured profile, e.g. the normalized signal S as function of position X in mm units, for the array according to embodiments 51 is compared with those obtained with an amorphous silicon flat panel 52 with 200µm spatial resolution. In the case of fields with this size, the amorphous silicon flat panel may be considered a reliable benchmark. The incident radiation field was generated by a 60Co radiation source. A good correspondence between the measured profile 51 for the device according to embodiments of the present invention and the benchmark detector 52 can be clearly observed.

## Claims

1. A dosimetry device (10) for determining a spatial distribution of a quantity of radiation incident on the dosimetry device, the dosimetry device comprising:
- a segmented electrode assembly (12) comprising an electrically non-conducting substrate (13) having a plurality of electrode elements (14) provided thereon, and
- an electrically conducting sheet (16) comprising a perimetral protrusion (17) arranged in mechanical contact with said segmented electrode assembly (14) such as to define at least one ionization chamber cavity (18) between the segmented electrode assembly (14) and the electrically conducting sheet (16), the at least one ionization chamber cavity (18) being filled with a fluid, and
- a voltage applying means (28) for applying a voltage difference between the electrically conducting sheet (16) and the plurality of electrode elements (14) and a routing means (25) for routing a plurality of ionization currents corresponding to the plurality of electrode elements (14) to a processing means.

2. The dosimetry device (10) according to claim 1, in which said segmented electrode assembly (12) comprises a printed circuit board having said plurality of electrode elements (14) defined thereon.

3. The dosimetry device (10) according to claim 2, in which said plurality of electrode elements (14) is printed on the printed circuit board using a carbon-based material.

4. The dosimetry device (10) according to any of the previous claims, in which said electrically conducting sheet (16) comprises a material with a low atomic number such as carbon , conductive plastic and/or aluminum.

5. The dosimetry device (10) according to any of the previous claims, in which the electrically conducting sheet (16) comprises at least one further protrusion (19) to further define the at least one additional ionization chamber cavity (18) such that the electrically conducting sheet (16) comprises a plurality of concave surface regions corresponding to the plurality of electrode elements (14).

6. The dosimetry device (10) according to any of the previous claims, in which the plurality of electrode elements (14) are arranged along at least a one dimensional or two dimensional regular grid on said electrically non-conducting substrate (13).

7. The dosimetry device (10) according to any of the previous claims, in which the plurality of electrode elements (14) extend into the at least one ionization chamber cavity (18) and the plurality of electrode elements (14) are adapted in shape for tailoring the electric field in said at least one ionization chamber cavity (18).

8. The dosimetry device (10) according to any of the previous claims, in which the segmented electrode assembly (12) comprises a plurality of guard electrodes (24), each guard electrode being arranged around a corresponding electrode element of the plurality of electrode elements (14).

9. The dosimetry device (10) according to any of the previous claims, in which:
- the plurality of electrode elements comprises a first plurality of electrode elements (31) provided on a first major surface of the electrically non-conducting substrate (13) and a second plurality of electrode elements (32) provided on a second major surface of the electrically non-conducting substrate (13),
- the perimetral protrusion (17) is arranged in mechanical contact with the first major surface of the electrically non-conducting substrate (13), and
- the dosimetry device further comprises a second electrically conducting sheet (36) comprising a perimetral protrusion arranged in mechanical contact with the second major surface of the electrically non-conducting substrate (13) such as to define at least one ionization chamber cavity between the segmented electrode assembly (14) and the electrically conducting sheet (16).

10. The dosimetry device (10) according to claim 9, in which the first plurality of electrode elements (31) and the second plurality of electrode elements (32) are arranged according to the same pattern on respectively the first major surface and the second major surface, and in which the device comprises means for electrically connecting each electrode of the first plurality of electrodes to the corresponding electrode of the second plurality of electrodes.

11. The dosimetry device (10) according to claim 9, in which the first plurality of electrode elements (31) and the second plurality of electrode elements (32) are arranged according to the same pattern, the first plurality of electrode elements (31) being spatially offset in the plane of the substrate with respect to the second plurality of electrode elements (32).

12. The dosimetry device (10) according to any of the previous claims, comprising :
at least one further segmented electrode assembly comprising a further electrically non-conducting substrate having a further plurality of electrode elements provided thereon, and at least one further electrically conducting sheet comprising a further perimetral protrusion arranged in mechanical contact with said at least one further segmented electrode assembly such as to define at least one further ionization chamber cavity between the at least one further segmented electrode assembly and the at least one further electrically conducting sheet.

13. A dosimetry system comprising a dosimetry device (10) according to any of the previous claims and a processing means (26) for receiving the plurality of ionization currents corresponding to the plurality of electrode elements (14) via the routing means (25), the processing means being adapted for determining a spatial distribution of radiation dose, radiation dose rate or radiation fluence corresponding to a radiation quantity incident on the dosimetry device (10) taking into account the plurality of ionization currents.

14. A method (80) for determining a spatial distribution of a quantity of radiation, the method comprising:
- applying (81) a voltage difference between a plurality of electrode elements (14) provided on an electrically non-conducting substrate (13) and an electrically conducting sheet (16) having a perimetral protrusion (17) arranged in mechanical contact with the electrically non-conducting substrate (13), the perimetral protrusion defining at least one ionization chamber cavity (18) between the electrically non-conducting substrate (13) and the electrically conducting sheet (16), and
- determining (82) a plurality of ionization currents corresponding to the plurality of electrode elements (14) generated by interaction of radiation within a fluid medium in said at least one ionization chamber cavity (18).

15. The method according to claim 14, further comprising positioning (83) the electrically non-conducting substrate (13) and the electrically conducting sheet (16) between a source of the radiation and a target, such that the radiation travels through the at least one ionization chamber cavity (18) prior to delivering the radiation to the target.
